# FASCICULE DE BREVET EUROPEEN

(11) **EP 0 575 230 B1**
(45) Date de publication et mention de la délivrance du brevet: **30.11.1994**
(21) Numéro de dépôt: 93401510.8
(22) Date de dépôt: 11.06.1993
(51) Int. Cl.: G06K 17/00, G06F 15/42, A61B 5/117

(54) **Système automatique d'impression d'un formulaire administratif médical**
System zum automatischen Drucken eines Medizinischen Verwaltungsformulars
Automatic printing system of a medical administrative form

(30) Priorité: 16.06.1992 FR 9207293
(43) Date de publication de la demande: 22.12.1993
(73) Titulaire: GEMPLUS CARD INTERNATIONAL, F-13420 Gémenos (FR)
(72) Inventeur: Lassus, Bruno, F-75116 Paris (FR); Sarat, Jean-Marc, F-75116 Paris (FR)
(74) Mandataire: Schmit, Christian Norbert Marie

(56) Documents cités:
- WO-A-91/02447
- WO-A-91/15817
- FR-A- 2 583 546
- US-A- 4 709 136
- PATENT ABSTRACTS OF JAPAN vol. 10, no. 38 (P-428)(2095) 14 Février 1986

## Description

La présente invention a pour objet un système automatique d'impression d'un formulaire administratif tel qu'une ordonnance médicale ou une feuille de remboursement d'un acte médical. Elle a pour but de simplifier les opérations de transcription des informations nécessaires à un formulaire médical afin de rendre plus lisibles les informations écrites tout en limitant les risques de fraude.

Actuellement il n'existe pas de dispositif permettant l'impression automatique des formulaires médicaux. On rappelle que sur un tel formulaire doivent figurer d'une part le nom du médecin ainsi que ses références d'habilitation à exercer la médecine. D'autre part, doivent figurer le nom du patient, si possible son adresse et éventuellement même son numéro d'identification auprès d'un organisme de couverture sociale. On connaît des systèmes à traitement de texte avec lesquel il serait possible de composer le texte d'un formulaire. Une fois le texte composé, il est possible de le faire imprimer par une imprimante raccordée à un microordinateur mettant en oeuvre le traitement de texte. Cependant un tel appareil présente l'inconvénient d'être relativement onéreux et encombrant sur le bureau du médecin. En outre, les contraintes de saisie au clavier des références des patients sont fastidieuses, de sorte que d'une manière générale de tels formulaires imprimés ne sont pas utilisés.

Un autre problème à résoudre est celui de la fraude qui résulte du vol des formulaires ou ordonnances pré-imprimés dont disposent les médecins. Les personnes mal intentionnées s'approprient de tels carnets d'ordonnances et rédigent pour leur compte des ordonnances leur permettant d'acquérir les médicaments interdits ou de bénéficier de remboursement illicites. Un système à traitement de texte, est peu protégé contre de tels tentatives, compte tenu que les fraudeurs peuvent avec lui éditer autant d'ordonnances qu'ils le désirent. US-A-4 709 136 divulgue un système de traitement de données médicales comportant deux cartes à puce (médecin/patient) qui sont introduit simultanément dans un lecteur. Pour des raisons de sécurité des données ne sont écrit que simultanément sur les deux cartes et peuvent être lues que si les codes de sécurité sont valables pour les deux cartes.

L'invention a pour objet de remédier à ces inconvénients tout en proposant un système beaucoup plus ergonomique et beaucoup plus simple, ayant par ailleurs toutes les fonctionnalités requises.

Le principe de l'invention consiste à disposer d'un lecteur de cartes à puce relié à une imprimante. Au moment de son branchement électrique le lecteur de carte à puce n'est pas configuré, n'est pas armé. Les médecins sont munis de cartes à puce de type médecin et les patients sont munis de cartes à puce de type patient. Pour élaborer une ordonnance avec le système, il faut d'une part configurer le lecteur en y introduisant d'abord une carte de médecin. Dans ce cas les informations relatives à ce médecin sont prélevées dans ces cartes à puce de médecin et stockées dans une première zone d'une mémoire volatile du lecteur. Lorsqu'un patient se présente, il donne sa carte à puce de type patient qui est introduite également dans l'appareil. A ce moment, les données relatives à ce patient sont transférées par l'appareil à l'imprimante qui les imprime, de même que les références du médecin, en des endroits adéquats. On montrera qu'en agissant de cette façon on peut avoir un appareil peu coûteux et susceptible de s'adapter facilement à toutes sortes de situations.

L'invention a donc pour objet un système automatique d'impression d'un formulaire médical, comportant les caractéristiques de la revendication 1.

L'invention sera mieux comprise à la lecture de la description qui suit et à l'examen des figures qui l'accompagnent. Celles ci ne sont données qu'à titre indicatif mais nullement limitatif de l'invention. Les figures montrent :
- Figure 1 : un système conforme à l'invention ;
- Figures 2 à 4 : un ensemble de programmes mis en oeuvre par le système de l'invention pour provoquer l'impression et garantir sa sécurité.

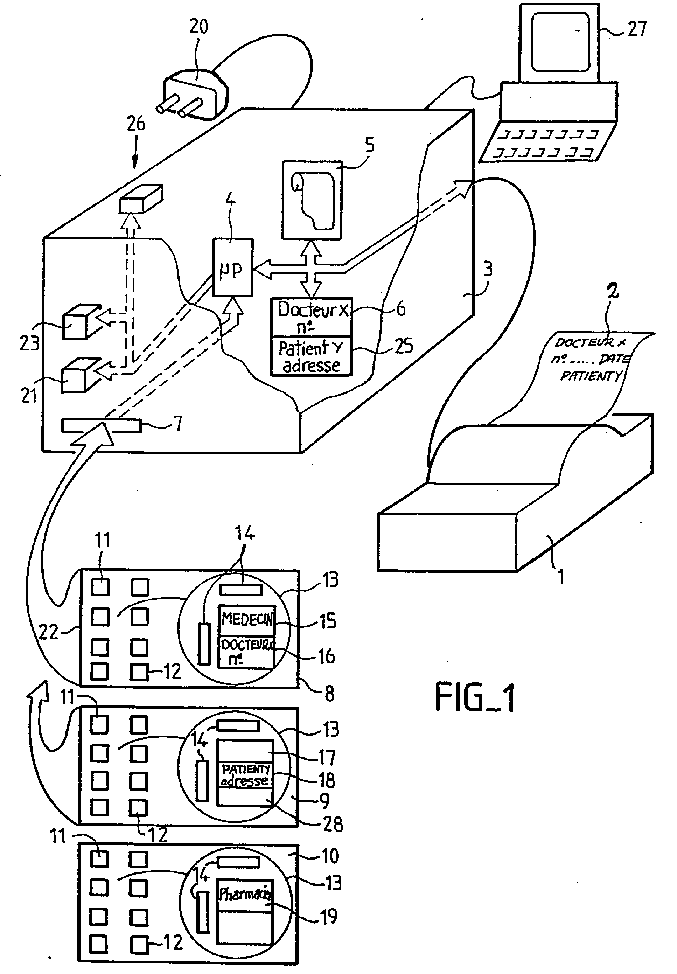

La figure 1 représente un système conforme à l'invention. Celui ci comporte un imprimante 1 destinée à imprimer sur des feuilles 2 des formulaires ou ordonnances. Cette imprimante est reliée à un lecteur 3 de cartes à puce. Le lecteur 3 est muni d'un microprocesseur 4, d'une mémoire programme 5 et, dans une mémoire volatile, d'au moins une première zone 6. Le microprocesseur 4 exécute les instructions contenues dans le programme de la mémoire 5 en vue d'imprimer sur l'ordonnance 2 des informations, en partie contenues dans la première zone de mémoire volatile 6. Le lecteur 3 comporte également une fente 7 par laquelle peuvent être introduites des cartes à puce tels que 8, 9 ou 10. Le système, dans sa version minimum, comporte au moins une carte à puce 8 dévolue à un médecin et une carte à puce 9 dévolue à un patient. Il y a autant de cartes à puce de médecin qu'il y a de médecins à équiper. Il y a autant de cartes à puce de patient qu'il y a de patients à équiper. Les cartes sont nominatives.

Dans l'utilisation, le lecteur 3 est placé chez le médecin, ainsi que l'imprimante 1. Chaque patient est muni de sa carte à puce de type patient. Les cartes de type médecin ou de type patient sont toutes les deux lisibles par le lecteur 3. Ces cartes à puce comportent d'une manière classique un jeu de connexions métalliques telles que 11 à 12 qui permettent au monde extérieur d'entrer en communication électrique avec une mémoire 13 électronique contenue dans la puce de la carte. Une carte 8 de type médecin comporte dans sa mémoire 13 un décodeur d'adresse 14 connecté aux cellules mémoires de la mémoire par des lignes de bit et des lignes de mot. La mémoire 15 comporte dans la carte médecin au moins deux zones non effaçables, par exemple réalisées avec des cellules mémoires de type EPROM. Une première zone 15, appelée ici l'en-tête, est relative à la qualité du titulaire de la carte 8 : à sa qualité de médecin. Une deuxième zone 16 comporte les informations relatives à son titulaire c'est à dire essentiellement le nom du médecin ainsi qu'éventuellement son numéro d'exercice professionnel.

La carte à puce 9 dévolue à un patient contient elle aussi dans sa mémoire 13 non volatile, un en-tête 17 et une zone 18 de stockage d'informations relatives à ce patient. Par exemple ces informations sont le nom du patient, ainsi que son adresse et éventuellement son numéro d'affiliation à un organisme de couverture sociale. Les entêtes 15 et 17 sont essentiellement différents l'un de l'autre. Leur codage binaire peut être sur un nombre quelconque de bits : par exemple même sur un seul bit, le bit 1 étant réputé le bit caractéristique des médecins, alors que le bit 0 serait caractéristique des patients. Dans la pratique, pour donner plus de souplesse au système, et dans une variante préférée, on créera également des cartes de type pharmacien ou autre, auquels cas l'en-tête comportera sur plusieurs bits une indication selon laquelle la carte est une carte de pharmacien ou autre.

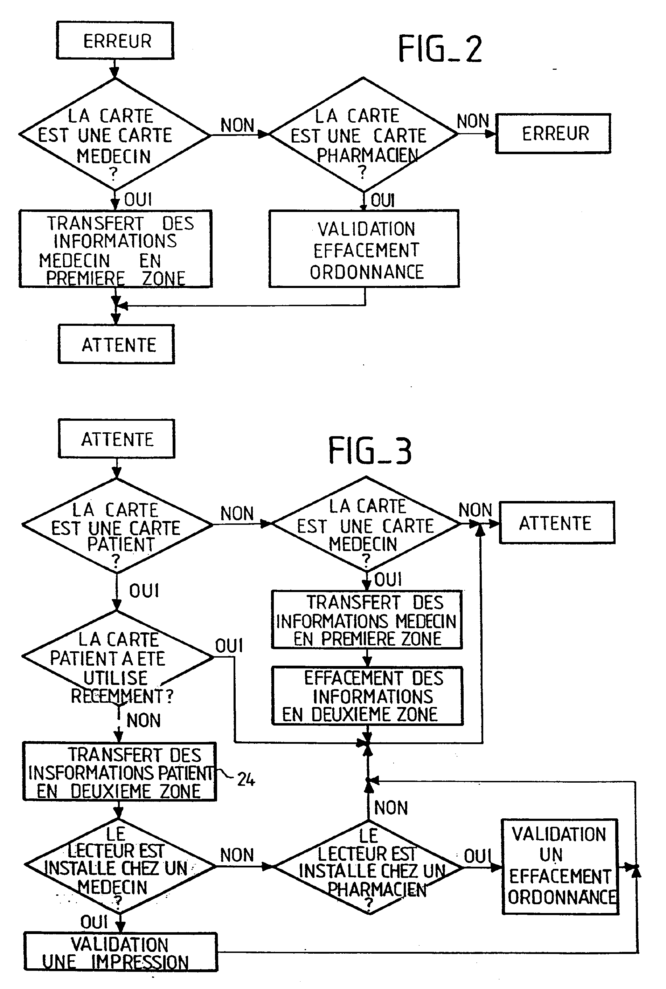

L'utilisation du système est la suivante. Chaque matin, le médecin met en service électriquement le lecteur 3 et l'imprimante 1. A ce moment, le lecteur démarre par un programme de démarrage contenu dans la mémoire 5. Ce démarrage peut être assimilable au démarrage d'un micro ordinateur. Puis il exécute une instruction par laquelle le lecteur 3 émet un signal d'erreur, ou d'alarme. Dans un exemple cette erreur est matérialisée par le clignotement d'une lampe 21 de couleur rouge, située par exemple en face avant du lecteur 3. Dans ce cas, figure 2, le lecteur est en état ERREUR. Puis le médecin introduit sa carte dans la fente 7. Arrivée au bout de cette fente 7, l'extrémité 22 de la carte y appuie sur un interrupteur fin de course (non représenté) de type connu qui déclenche l'exécution d'un premier programme de test de la carte.

Le programme de test a principalement pour objet de déterminer si la carte est une carte de médecin. En pratique, le contenu de la mémoire 13 de la carte 8 va être lu par le lecteur 3. Le contenu de l'en-tête 15 va être comparé à un paramètre préenregistré dans le programme de la mémoire 5. S'il y a concordance de l'en-tête et du paramètre, le test est positif, et on transfère dans la première zone volatile 6 du lecteur 3 les informations relatives au médecin, contenues dans la zone 16 de sa carte à puce 8. Pendant ce transfert, la lampe 21 reste allumée fixe. Une fois que ceci est effectué, le microprocesseur 4 du lecteur 3 se place dans une situation d'attente. Cette situation d'attente permet au microprocesseur 4 de pointer tout simplement une autre instruction, ATTENTE, du programme contenue dans la mémoire 5. En fin de test, quand il est positif, la lampe rouge 21 s'est éteint. Par contre, si la carte 8 n'a pas été reconnue par le lecteur comme étant une carte de médecin, cela conduit à la situation de départ : ERREUR.

Lorsqu'un patient se présente, normalement le lecteur 3 est en attente. On introduit la carte du patient. Un autre test, lui aussi comparant la valeur de l'en-tête 17 à un paramètre de type patient contenu dans le programme 5 permet de reconnaître ou non la carte à puce d'un patient. Pendant cette autre opération de test, une deuxième lampe 23, de couleur verte de préférence, clignote. Lorsque l'autre test est terminé, et s'il est positif, la lampe verte reste allumée. Dans ce cas, on aura transféré avec une instruction 24 du programme, figure 3, les informations contenues dans la zone 18 de la mémoire 13 de la carte 9 dans une deuxième zone volatile 25 de la mémoire volatile du lecteur 3. La lumière verte allumée vaut validation de l'impression.

Lorsque le médecin veut imprimer le formulaire ou l'ordonnance, et lorsque la lampe 23 verte est allumée, il peut appuyer sur un bouton de commande 26 ce qui constitue un ordre final de déclenchement de l'impression. Cet ordre 26 fait exécuter au microprocesseur 4 une partie du programme 5 consistant à imprimer réellement l'ordonnance. Ce programme 5 est de type classique. Il comporte essentiellement des instructions de mise en place du papier dans l'imprimante, d'avance et de désignation de caractères, de retour de chariot, et d'éjection du papier à l'issue. Bien entendu, ce programme comporte, comme tout traitement de texte, l'envoi de préférence sous une forme de code ASCII des informations relatives au médecin et au patient et qu'il y a lieu d'imprimer sur l'ordonnance 2. La deuxième zone 25 n'est pas indispensable si on choisit d'autoriser l'impression alors que la carte patient est dans le lecteur 3. L'impression peut alors être directe. En variante, l'insertion de la carte dans le lecteur provoque à l'issue l'impression. Ce qui permet de simplifier l'appareil en supprimant le bouton 26. Dans ce cas l'insertion de la carte provoque en séquence la reconnaissance de la carte patient, le transfert en zone volatile 25, puis l'impression réelle de l'ordonnance.

Lorsque le bouton 26 a été appuyé une fois, la lampe verte s'éteint, et on ne peut normalement pas éditer une autre ordonnance avec introduction de la même carte patient. Le formulaire imprimé peut néanmoins comporter plusieurs pages. Le but de cette impression unique est d'éviter que des fraudeurs, des drogués, établissent à leur nom plusieurs ordonnances en utilisant ce système. Une fois qu'une ordonnance a été imprimée, normalement on bloque le système. Dans ce but, si on réintroduit la carte du patient à nouveau dans le lecteur, elle va être à nouveau lue. Le programme contenu dans la mémoire 5 comporte une suite d'instructions pour vérifier si la carte du patient vient d'être utilisée récemment. Les vérifications de cette utilisation récente peuvent prendre diverses formes. Par exemple, au moment du chargement des informations relatives au patient dans la deuxième zone non volatile 25, on peut regarder s'il s'est écoulé un temps suffisant (par exemple au moins plusieurs heures) entre la première et la seconde présentation. Si la seconde présentation est trop proche de la première et si le nom à inscrire est le même que le précédent, on refuse de valider l'impression et le lecteur se remet en attente.

Pour permettre cependant, à un médecin qui voudrait le faire, de délivrer pour un même patient deux ordonnances ou deux formulaires, on a prévu que ce médecin peut réintroduire sa carte personnelle dans le lecteur une deuxième fois. Dans ce cas, n'étant pas reconnue comme une carte patient, cette carte va être reconnue dans un test suivant comme une carte médecin. Cette reconnaissance va pouvoir provoquer, comme précédemment, le transfert des informations relatives au médecin dans la première zone 6 de la mémoire volatile du lecteur 3. Mais elle provoque également l'effacement des informations contenues dans la deuxième zone 25 de la mémoire volatile. Après cette réintroduction de la carte à puce médecin on peut réintroduire une fois de plus la même carte à puce du patient. Cette procédure peut bien entendu être réitérée.

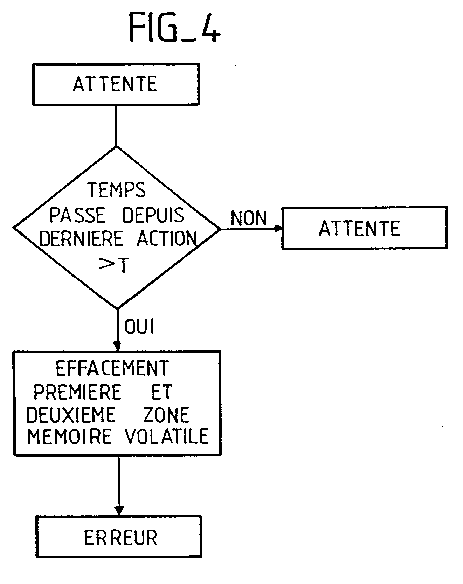

Au cas où le médecin oublierait de débrancher son lecteur en quittant son cabinet le soir, le programme contenu dans la mémoire 5 comporte également un test périodique, figure 4, par lequel on teste la durée qui sépare le temps présent du temps où on a effectué la dernière opération. Ceci est par exemple possible avec une indication de temps stockée en regard des zones 6 et 25 de la mémoire volatile. Ces informations de temps sont naturellement disponibles du fait que le microprocesseur comporte une horloge temporelle du même type que celle qu'on trouve dans une montre à quartz. Tant que cette durée est inférieure à une durée T choisie on maintient le lecteur en attente. Dès qu'elle dépasse cette durée T, on met le lecteur en erreur, en effaçant au préalable le contenu des zones 6 et 25 de la mémoire volatile. Dans ce cas la lampe rouge 21 se remet à clignoter.

Dans une autre solution, beaucoup plus perfectionnée, le lecteur 3 est relié à un microordinateur 27, muni d'un clavier, d'un écran, et d'une mémoire de masse, par exemple un disque dur. Dans cette mémoire de masse on a stocké la monenclature de tous les médicaments prescriptible, ainsi que tous les modes de prises possibles et conseillables. Avec le clavier, le médecin peut faire apparaître sur son écran une liste de produits prescriptibles particuliers et sélectionner ceux qu'il veut prescrire avec la posologie. Il peut injecter ces informations dans le lecteur 3 de manière à ce qu'elles soient également imprimées sur le bas de l'ordonnance. En variante, ce sont les informations des mémoires volatiles 6 et 25 qui sont transmises au microordinateur, celui ci se chargeant de l'impression de l'ordonnance avec une imprimante qui lui est raccordée.

Dans cette autre solution le lecteur 3 se met sous l'autorité du microordinateur 27. Celui-ci peut aussi être capable d'écrire le contenu de l'ordonnance dans la carte à puce du patient 10, dans une mémoire 28 qui est également du type non volatile, mais effaçable électriquement de préférence. Par exemple cette mémoire supplémentaire 28 est du type EEPROM. Avec une telle carte le patient peut alors se rendre chez un pharmacien qui lui aussi possède un lecteur 3 également raccordé à un microordinateur 27 pour visualiser l'ordonnance électronique et *vérifier que, par ailleurs, rien n'a été* falsifié entre le contenu électronique stocké dans la carte à puce 9 et l'ordonnance imprimée 2 qu'on lui montre.

Le pharmacien agit de même que le médecin : tous les matins, il introduit sa carte à puce 10 dans son lecteur 3. Celui ci reconnaît alors, figure 2, que la carte est une carte de pharmacien (l'en-tête est spécifique de cette profession). Dans ces conditions la partie du programme contenu dans la mémoire 5 et spécifique à cette profession provoque une validation potentielle de l'effacement des ordonnances. Par la suite, lorsque un patient muni de sa carte à puce 10 dans laquelle est enregistrée l'ordonnance se présente chez ce pharmacien, cette carte 10 est reconnue par le lecteur 3 du pharmacien. Du fait que ce lecteur est installé justement chez un pharmacien on va pouvoir effacer l'ordonnance électronique. Cette autorisation est matérialisée par le maintien allumé en permanence de la lampe verte 23. Lorsque le pharmacien appuie sur le bouton 26, le microprocesseur 4 met en oeuvre un programme contenu dans la mémoire ayant pour but d'effacer l'ordonnance électronique stockée dans la partie 28 électriquement programmable de la mémoire de la carte à puce 9. Ceci a trois avantages, d'une part cela interdit à quelqu'un de malveillant de se ravitailler à plusieurs pharmacies à partir de la même ordonnance électronique. D'autre part, cela simplifie les opérations de gestion des ordonnances dans les mémoires des cartes à puce qui sont malgré tout de capacité assez réduite. Enfin le pharmacien peut directement utiliser ces informations pour gérer ses stocks.

A titre de perfectionnement supplémentaire le lecteur 3 comporte une mémoire non volatile pour stocker une copie des ordonnances délivrées. Une partie du programme contenu dans la mémoire 5 permet par ailleurs, à l'aide du microordinateur 27, d'effectuer des travaux statistiques, journaliers, mensuels ou annuels.

Plutôt que de ne définir que deux catégories de praticien, on peut en définir plusieurs autres, par exemple celle des kinésithérapeutes, des laboratoires d'analyse médicale, ou parmi même les médecins, celles concernant des spécialités. 11 peut être attaché à ces spécialités, avec les voyants 21 et 23 et avec le bouton 26, des fonctionnalités spécifiques tendant à écrire ou effacer d'autres informations dans la carte. Par exemple, on peut imaginer que, chez un kinésithérapeute, chacune des dix séances prescrites par le médecin sera oblitérée dans la carte à chaque fois.

## Revendications

1. Système automatique d'impression d'un formulaire (2) médical, comportant
- une imprimante (1) pour imprimer les formulaires,
- un lecteur (3) de cartes (8-10) à puce (13) muni d'un microprocesseur (4), d'une mémoire programme (5) pour contenir des instructions d'impression exécutables par le microprocesseur, et d'au moins une première zone (6) dans une mémoire volatile, ce lecteur étant connecté à cette imprimante,
- une carte (8) à puce dévolue à un médecin contenant, dans sa mémoire non effaçable, un en-tête (15) représentatif de la qualité de médecin de son titulaire et une zone (16) de stockage d'informations relatives à ce médecin, ces informations étant destinées à être imprimées sur le formulaire après avoir été stockées (6) dans la première zone de la mémoire volatile,
- une carte à puce (9) dévolue à un patient contenant un en-tête (17) représentatif de la qualité de patient de son titulaire et une zone (18) de stockage des informations relatives à ce patient et destinées à être imprimées sur le formulaire.

2. Système selon la revendication 1, caractérisé en ce que le lecteur comporte
- un circuit (21) pour signaler, lors de sa mise sous tension, que la première zone mémoire de sa mémoire volatile est vide, et pour signaler que cette première zone mémoire de sa mémoire volatile est remplie avec les informations relatives au médecin après que celui-ci aura introduit sa carte à puce dans ce lecteur.

3. Système selon la revendication 1 ou la revendication 2, caractérisé en ce que le lecteur comporte
- un circuit (23) pour signaler, lors de l'introduction d'une carte à puce d'un patient, le caractère acceptable de cette carte à puce, et pour signaler la lecture dans cette carte à puce des informations relatives à ce patient et destinées à être imprimées sur le formulaire.

4. Système selon la revendication 3, caractérisé en ce que le lecteur comporte au moins une deuxième zone (25) dans la mémoire volatile, pour mémoriser les informations relatives à ce patient et destinées à être imprimées sur le formulaire.

5. Système selon l'une quelconque des revendications 1 à 4, caractérisé en ce que le lecteur comporte
- un circuit (26) muni d'un bouton d'action pour provoquer l'impression du formulaire en imprimant en des lieux déterminés de celle-ci des informations relatives au médecin et au patient.

6. Système selon la revendication 5, caractérisé en ce que le circuit pour provoquer l'impression du formulaire comporte
- un circuit pour empêcher l'impression si les informations lues dans la carte à puce du patient sont les mêmes que des informations préalablement mémorisées dans la deuxième zone (25) de la mémoire volatile.

7. Système selon l'une quelconque des revendications 1 à 6, caractérisé en ce que le lecteur comporte
- un circuit pour effacer le contenu de la première zone de la mémoire volatile après une certaine durée (T), de préférence consécutive à une dernière utilisation du système.

8. Système selon l'une quelconque des revendications 1 à 7, caractérisé en ce que le lecteur comporte
- une horloge temps réel pour mémoriser le temps et éventuellement imprimer la date sur le formulaire.

9. Système selon l'une quelconque des revendications 1 à 8, caractérisé en ce que le lecteur comporte fonctionnellement
- une mémoire non volatile dans laquelle sont répertoriés des médicaments prescriptibles,
- un écran de visualisation pour visualiser ces médicaments prescriptibles,
- un clavier pour désigner un médicament à prescrire et sa posologie,
- et des circuits pour imprimer ces prescriptions sur le formulaire.

10. Système selon la revendication 9, caractérisé en ce que le lecteur comporte
- un circuit d'écriture pour écrire, dans une partie effaçable (28) de la mémoire de la carte à puce du patient, le contenu du formulaire.

11. Système selon la revendication 10, caractérisé en ce qu'il comporte
- une carte à puce (10) dévolue à un praticien contenant, dans sa mémoire non effaçable, un en-tête représentatif de la qualité de praticien de son titulaire, et
- dans le lecteur, un circuit (26) d'effacement pour effacer, dans la partie effaçable de la mémoire de la carte à puce du patient, le contenu du formulaire après reconnaissance de cet en-tête de praticien par le lecteur.

12. Système selon l'une quelconque des revendications 1 à 11, caractérisé en ce que le lecteur comporte
- une mémoire non volatile dans laquelle sont stockées toutes les ordonnances prescrites,
- un compteur pour compter ces ordonnances et pour éventuellement imprimer sur le formulaire une information relative à l'état de ce compteur, ou pour établir des statistiques.

13. Système selon l'une quelconque des revendications 1 à 12 caractérisé en ce qu'il comporte un automatisme pour imprimer un formulaire dès que la carte patient y est introduite.

## Patentansprüche

1. System zum automatischen Drucken eines medizinischen Verwaltungsformulars (2), mit:
- einem Drucker (1) zum Drucken der Formulare,
- einem Lesegerät (3) für Karten (8 - 10), die einen mit einem Mikroprozessor (4) versehenen Chip (13), einen Programmspeicher (5) zur Aufnahme von Druckinformationen, die durch den Mikroprozessor ausführbar sind und wenigstens einen ersten Bereich (6) in einem flüchtigen Speicher aufweisen, wobei das Lesegerät mit dem Drucker verbunden ist,
- einer einem Arzt zugeordneten Chipkarte (8), die in ihrem nichtlöschbaren Speicher eine Kopfleiste (15) aufweist, welche der Arztqualifikation ihres Inhabers entspricht und einen Bereich (16) zur Speicherung von diesem Arzt zugeordneten Informationen aufweist, wobei diese Informationen nach der Speicherung (6) im ersten Speicherbereich des flüchtigen Speichers auf dem Formular ausgedruckt werden sollen und
- einer einem Patienten zugeordneten Chipkarte (9), die eine Kopfleiste (17) aufweist, welche der Patientenqualifikation ihres Inhabers entspricht und einen Bereich (18) zur Speicherung von diesem Patienten zugeordneten Informationen aufweist, wobei diese Informationen auf dem Formular ausgedruckt werden sollen.

2. System nach Anspruch 1, dadurch gekennzeichnet, daß das Lesegerät aufweist:
- einen Schaltkreis (21) um anzuzeigen, daß während des Anlegens einer Spannung der erste Speicherbereich des flüchtigen Speichers leer ist und um anzuzeigen daß dieser erste Speicherbereich des flüchtigen Speichers mit den Arzt betreffenden Informationen belegt ist, nachdem dieser seine Chipkarte in das Lesegerät eingeführt hat.

3. System nach Anspruch 1 oder Anspruch 2, dadurch gekennzeichnet, daß das Lesegerät aufweist
- einen Schaltkreis (23) um anzuzeigen während des Einführens einer Chipkarte eines Patienten die akzeptable Eigenschaft dieser Chipkarte und um anzuzeigen während des Auslesens dieser Chipkarte die dem Patienten zugeordneten Informationen, die auf dem Formular ausgedruckt werden sollen.

4. System nach Anspruch 3, dadurch gekennzeichnet, daß das Lesegerät wenigstens einen zweiten Bereich (25) im flüchtigen Speicher aufweist zur Speicherung von den Patienten betreffenden Informationen, welche auf dem Formular ausgedruckt werden sollen.

5. System nach einem der Ansprüche 1 bis 4, dadurch gekennzeichnet, daß das Lesegerät aufweist
- einen Schaltkreis (26) mit einem Betätigungsknopf, welcher den Druck des Formulars bewirkt indem an vorgegebenen Stellen des Formulars die den Arzt und den Patienten betreffenden Informationen gedruckt werden.

6. System nach Anspruch 5, dadurch gekennzeichnet, daß der Schaltkreis zur Durchführung des Drucks des Formulars aufweist
- einen Schaltkreis der das Ausdrucken unterdrückt wenn die in der Chipkarte des Patienten enthaltenen Informationen die gleichen sind wie die vorher im zweiten Bereich (25) des flüchtigen Speichers gespeicherten Informationen.

7. System nach einem der Ansprüche 1 bis 6, dadurch gekennzeichnet, daß das Lesegerät aufweist
- einen Schaltkreis zum Löschen des Inhalts des ersten Bereich des flüchtigen Speichers nach einer bestimmten Zeitdauer (T), die sich vorzugsweise anschließt an den letzten Einsatz des Systems.

8. System nach einem der Ansprüche 1 bis 7, dadurch gekennzeichnet, daß das Lesegerät aufweist
- eine Echtzeituhr zur Speicherung der Zeit und um gegebenenfalls das Datum auf dem Formular auszudrucken.

9. System nach einem der Ansprüche 1 bis 8, dadurch gekennzeichnet, daß das Lesegerät wirkungsmäßig aufweist
- einen nicht-flüchtigen Speicher in dem verschreibungspflichtige Medikamente enthalten sind,
- einen Bildschirm zur Darstellung der verschreibungspflichtigen Medikamente,
- eine Tastatur um ein zu verschreibendes Medikament und dessen Dosierung auszuwählen und
- Schaltkreise um diese Verschreibungen auf dem Formular auszudrucken.

10. System nach Anspruch 9, dadurch gekennzeichnet, daß das Lesegerät aufweist
- einen Schreibschaltkreis um in einem löschbaren Bereich (28) des Speichers der Chipkarte des Patienten den Inhalt des Formulars einzuschreiben.

11. System nach Anspruch 10, dadurch gekennzeichnet, daß es aufweist
- eine einem Praktiker zugeordnete Chipkarte (10), welche in ihrem nichtlöschbaren Speicher eine Kopfleiste aufweist, welche der Praktikerqualifikation ihres Inhabers zugeordnet ist und
- im Lesegerät einen Löschschaltkreis (26) aufweist, um im löschbaren Bereich des Speichers der Chipkarte des Patienten den Inhalt des Formulars nach Erkennung der Kopfleiste des Praktikanten durch das Lesegerät zu löschen.

12. System nach einem der Ansprüche 1 bis 11, dadurch gekennzeichnet, daß das Lesegerät aufweist
- einen nicht-fließenden Speicher in welchem sämtliche Verschreibungen gespeichert sind,
- einen Zähler zur Zählung der Verschreibungen und um gegebenenfalls auf dem Formular eine Information auszudrucken bezüglich des Zustandes dieses Zählers oder um Statistiken zu erstellen.

13. System nach einem der Ansprüche 1 bis 12, dadurch gekennzeichnet, daß es eine Automatik aufweist um ein Formular auszudrucken sobald die Patientenkarte darin eingeführt worden ist.

## Claims

1. An automatic system for printing a medical form (2), comprising
- a printer (1) for printing forms,
- a reader (3) of smart cards (8-10) with chip (13) provided with a microprocessor (4), with a program memory (5) in order to contain printing instructions executable by the microprocessor, and with at least one first zone (6) in a volatile memory, this reader being connected to this printer,
- a smart card (8) reserved for a doctor containing, in its non-erasable memory, a header (15) representative of its holder's capacity of doctor and a zone (16) for storage of data relating to this doctor, these data being intended to be printed on the form after having been stored (6) in the first zone of the volatile memory,
- a smart card (9) reserved for a patient containing a header (17) representative of its holder's capacity of patient and a zone (18) for storage of the data relating to this patient and intended to be printed on the form.

2. The system according to Claim 1 characterised in that the reader comprises
- a circuit (21) for signalling, when it is energised, that the first memory zone of its volatile memory is empty, and for signalling that this first memory zone of its volatile memory is filled with the data relating to the doctor after the latter will have inserted his smart card into this reader.

3. The system according to Claim 1 or Claim 2, characterised in that the reader comprises
- a circuit (23) for signalling, when a patient's smart card is inserted, the acceptable nature of this smart card, and for signalling the reading from this smart card of the data relating to this patient and intended to be printed on the form.

4. The system according to Claim 3, characterised in that the reader comprises at least one second zone (25) in the volatile memory, for memorising the data relating to this patient and intended to be printed on the form.

5. The system according to any of Claims 1 to 4, characterised in that the reader comprises
- a circuit (26) provided with an action button for bringing about the printing of the form by printing in specified places of the latter data relating to the doctor and to the patient.

6. The system according to Claim 5, characterised in that the circuit for bringing about the printing of the form comprises
- a circuit for preventing the printing if the data read from the patient's smart card are the same as the data previously memorised in the second zone (25) of the volatile memory.

7. The system according to any of Claims 1 to 6, characterised in that the reader comprises
- a circuit for erasing the content of the first zone of the volatile memory after a certain time (T), preferably consecutive to a last use of the system.

8. The system according to any of Claims 1 to 7, characterised in that the reader comprises
- a real time clock for memorising the time and possibly for printing the date on the form.

9. The system according to any of Claims 1 to 8, characterised in that the reader comprises functionally
- a non-volatile memory in which are indexed prescriptible medicaments,
- a display screen for displaying these prescriptible medicaments,
- a keyboard for designating a medicament to be prescribed and its dosage,
- and circuits for printing these prescriptions on the form.

10. The system according to Claim 9, characterised in that the reader comprises
- a writing circuit for writing, in an erasable part (28) of the memory of the patient's smart card, the content of the form.

11. The system according to Claim 10, characterised in that it comprises
- a smart card (10) reserved for a practitioner containing, in its non-erasable memory, a header representative of its holder's capacity of practitioner, and
- in the reader, an erasure circuit (26) for erasing, from the erasable part of the memory of the patient's smart card, the content of the form after recognition of this practitioner's header by the reader.

12. The system according to any of Claims 1 to 11, characterised in that the reader comprises
- a non-volatile memory in which are stored all the prescriptions prescribed,
- a counter for counting these prescriptions and possibly for printing on the form an information relating to the state of this counter, or for compiling statistics.

13. The system according to any of Claims 1 to 12 characterised in that it comprises an automatic system for printing a form as soon as the patient card is inserted into it.
